# EUROPEAN PATENT SPECIFICATION

(11) **EP 1 957 152 B1**
(45) Date of publication and mention of the grant of the patent: **08.06.2011**
(21) Application number: 05810017.3
(22) Date of filing: 30.11.2005
(51) Int. Cl.: A61N 1/05

(54) **ENDOCARDIAL LEAD**
ENDOKARDIALE LEITUNG
DÉRIVATION ENDOCARDIAQUE

(43) Date of publication of application: 20.08.2008
(73) Proprietor: St Jude Medical AB, 17584 Järfälla (SE)
(72) Inventor: HILL, Rolf, S-175 55 Järfälla (SE)
(86) International application number: PCT/SE2005/001810
(87) International publication number: WO 2007/064263

(56) References cited:
- WO-A-2007/053065
- US-A- 5 456 708
- US-A- 5 837 006
- US-A1- 2005 070 984
- US-B1- 6 687 550
- US-B1- 6 819 959

## Description

The invention relates to a device for extending and retracting of a helix from a distal end of a cardiac stimulator lead, of the type used for transmitting electrical signals from a pacemaker to a position inside a heart for stimulating the heart activity, wherein the helix is used for attaching the cardiac stimulator lead to a heart wall by screw rotating the helix into the heart wall, comprising a tip tube, having on the inside a post which protrudes between adjacent coils of the helix, and a shaft on which the helix is mounted, such that when rotating the shaft by means of a rotary member, the helix as well as the shaft will be screw rotated outward or inward in the tip tube by engagement between the helix and the post.

The invention also relates to a cardiac stimulator lead comprising such a device.

### Background of the invention

When screw rotating a helix of a cardiac stimulator lead, for extending it from the distal end of the tip tube to attach the cardiac stimulator lead to the heart wall, it is essential that the helix will not be extended too far out from the tip tube since the helix then may penetrate through the heart wall. It is also essential that the helix may be retracted again if, for any reason, it is desirable to detach the cardiac stimulator lead if necessary. This may be difficult or even impossible if the helix has been extended so far such that the helix coils have been disengaged from the post. It is also a risk that the post and the shaft can get jammed together, if the helix is extended so far such that the post and the shaft will meet, which will make it difficult or impossible to retract the helix.

In the prior art it is known to arrange a separate stop member in the proximal end of the shaft, which will restrict the maximum extension of the shaft and helix by abutment against a holder holding the rotary shaft. However, the developments go towards thinner cardiac stimulator leads, preferably less than 3 mm outside diameter. It is then a need for reducing the number of small components, such as the stop member, since these small components will be very difficult to handle during assembling in production and also expensive to produce.

US 2005/0070984 discloses a cardiac stimulator lead having a helix attached to shaft mounted in a tip tube of the cardiac stimulator. Guides extend from the tip tube between helix coils to translate a rotation of the helix and shaft into a longitudinal movement. Outward movement of the helix is stopped once a shoulder of the shaft abuts an inwardly protruding portion of the tip tube.

US 5,837,006 discloses a cardiac stimulator lead having a helix attached to a shaft mounted in a tip tube of the cardiac stimulator. A guide mechanism is provided in the distal lead portion to translate a rotation of the helix and shaft into a longitudinal movement. Outward movement of the helix is stopped once the distal surface of the shaft abuts the guide mechanism.

US 5,456,708 discloses a cardiac stimulator lead having a helix attached to a shaft mounted in a tip tube of the cardiac stimulator. A post projects inwardly from the tip tube between adjacent helix coils to translate a rotation of the helix and shaft into a longitudinal movement. Outward movement of the helix is stopped once a shoulder of the shaft abuts an inwardly protruding seal assembly of the tip tube.

US 6,687,550 discloses a cardiac stimulator lead having a helix attached to a shaft mounted in a tip tube of the cardiac stimulator. A post projects inwardly from the tip tube between adjacent helix coils to translate a rotation of the helix and shaft into a longitudinal movement. Outward movement of the helix is stopped once a tubular conductive coupling spring becomes too compressed between a shoulder of the shaft and an inwardly protruding element of the tip tube.

US 6,819,959 discloses a cardiac stimulator lead having a helix attached to a shaft mounted in a tip tube of the cardiac stimulator. The shaft comprises an outwardly protruding follower member slidably engaged with a spiral track member of the tip tube to translate a rotation of the helix and shaft into a longitudinal movement. Outward movement of the helix is stopped once a compression spring becomes fully compressed between a shaft shoulder and a planar bulkhead member extending transversely of the longitudinal axis of the lead.

WO 2007/053065 discloses a cardiac stimulator lead having a helix attached to a shaft mounted in a tip tube of the cardiac stimulator. A post projects inwardly from the tip tube between adjacent helix coils to translate a rotation of the helix and shaft into a longitudinal movement. Outward movement of the helix is stopped once the distal end of the shaft abuts the post.

### Summary of the invention

The present invention is defined in claim 1. Prefered features of the present invention are provided in the dependent claims.

It is an object of the invention to eliminate or reduce the drawbacks according to the prior art and provide a device, for extending and retracting of a helix from a distal end of a cardiac stimulator lead, which is able to restrict the extension of the helix without the risk of the helix to get jammed or without the need for any separate stop member. At least this object is achieved by a device according to claim 1.

The invention is thus based on the understanding that this object may be achieved by a shaft, for holding of the helix, which is formed with a stop surface.

Within this general inventive idea, the invention may be implemented in many different ways. However, the stop surface must be oriented such that its extension has at least one component perpendicular to the direction in which the shaft is moved when rotated for extending the helix. Also, the shape of the stop surface may be any arbitrary, appropriate shape being able to function as a stop surface, e.g. a curved or a plane surface.

In a hereinafter described and in the drawings illustrated embodiment of the invention, the stop surface is a plane surface and oriented in a plane which is parallel to a radial direction and the longitudinal directions of the shaft. However, the invention is not restricted to this orientation. The stop surface could for example also be oriented in a plane which is parallel to a radial direction but has a small angle, corresponding to the pitch angle of the helix, to the longitudinal direction, such that the stop surface is slightly turned towards the tip of the helix, i.e. in the direction in which the stop surface is moved when screw rotating the helix to an extended position. In reality the actual stop surface may deviate a rather great deal from the latter orientation. However, in order to eliminate the risk for jamming between the stop surface and the post, the deviation in relation to the orientation perpendicular to the pitch angle, should be less than 45° and preferably less than 30° in relation to a radial direction as well as the direction perpendicular to the screw pitch of the helix.

Within the scope of the invention, the device may be formed in many different ways. For example, in the described embodiment the stop surface is formed on the shaft in the area between the two most proximal coils of the helix. The helix is attached to the shaft by way of a groove, which is formed in an enlarged portion of the shaft and which has a shape corresponding to the helix. However, both of these features as well as many others, may be performed in different ways as will be evident for anyone skilled in the art.

As used herein, the term distal relates to a position or a direction closer to the outermost end of the cardiac stimulator lead, which is intended to be attached to the heart wall, whereas the term proximal relates to a position or a direction further away from the outermost end of the cardiac stimulator lead.

### Brief description of the drawings

In a hereinafter described exemplified embodiment of the invention, reference is made to the accompanying drawings, in which:
- Fig 1: is a partly cut through perspective view of a tip tube, located in the distal end of a cardiac stimulator lead, having the helix in a retracted position;
- Fig 2: is a perspective view according to fig 1 with the helix in an extended position;
- Fig 3: is a perspective view of the assembled helix and shaft mounted in a holder; and
- Fig 4: is a perspective view of the shaft.

### Detailed description of an embodiment of the invention

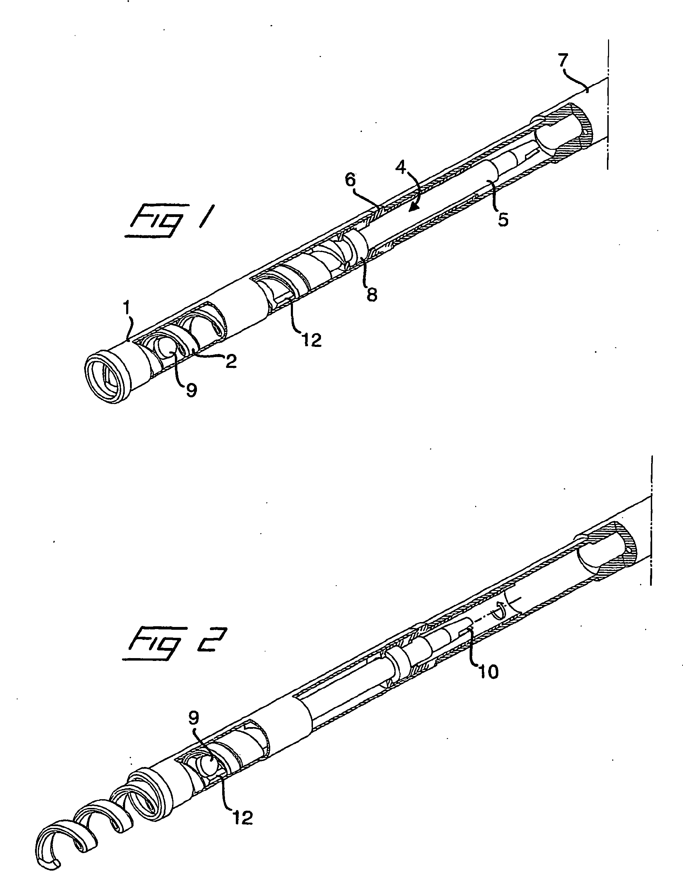

Reference is first made to figs 1 and 2, in which a distal end of a cardiac stimulator lead is shown in a partly cut through perspective view. The cardiac stimulator lead comprises a tip tube 1 in which a helix 2 is accommodated. The helix has a cork screw form and is in its proximal end attached to an enlarged helix holding portion 3 in the distal end of a shaft 4. The proximal portion of the shaft functions as a guiding portion 5, which is rotating and sliding supported in a holder 6 mounted to the proximal end of the tip tube. Besides a guiding function for the shaft 4, the holder 6 is adapted to provide a sealing around the shaft to prevent fluids from coming in from the tip tube into the holder and further into the cardiac stimulator lead, which is indicated at 7. The sealing effect is accomplished by a seal 8 between the holder and the shaft.

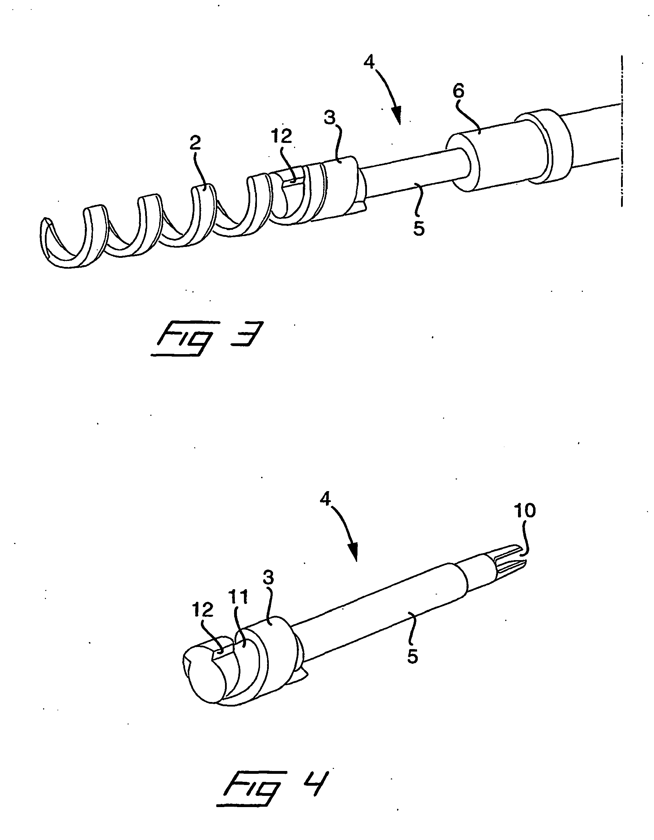

A post 9 protrudes from the inner surface of the tip tube 1 into a gap between two adjacent coils of the helix 2. By engagement between the helix coils and the post, the helix 2 and the holder 4 is caused to be extended or retracted when rotating the holder. This is illustrated in fig 2 where the helix 2 is extended out from the distal end of the tip tube 1 until the post 9 abuts against a stop surface 12. The rotation of the shaft 4 may be performed by any suitable means, such as a flexible torque transferring wire (not shown), which can be inserted through the cardiac stimulator lead and be moved into engagement with an engagement formation 10 in the proximal end of the shaft 4, as is best seen in fig 4.

Now reference is made to figs 3 and 4, wherein fig 3 shows the assembled helix 2 and shaft 4 inserted with the guide portion 5 of the shaft into the holder 6. The helix is attached to the shaft by way of a groove 11, as is best seen in fig 4 where the shaft 4 is illustrated separately. The groove 11 has a shape that essentially corresponds to the shape of the helix, such that a few coils of the helix may be threaded onto the shaft as is shown in fig 3. By removing a portion of the enlarged helix holding portion 3, a stop surface 12 is formed between the two most proximal coils of the helix. The stop surface, in the embodiment illustrated, is oriented in a plane which is parallel to a radial direction as well as the longitudinal direction of the shaft. I.e. the stop surface is oriented in a radial plane of the shaft.

In the view of fig 2, the helix is shown in an extended position in which the helix is extended out from the distal end of the tip tube. In this position, the stop surface 12 is in abutment with the post 9, which is positioned on the inner surface of the wall of the cut away tip tube 1, by screw rotation of the shaft 4. Accordingly, the helix is in this position restricted from extending any further out from the tip tube, however, without any risk for jamming of the post 9 between the helix (2) and the outer end face of the helix holding portion 3.

## Claims

1. A cardiac stimulator lead (7), of the type used for transmitting electrical signals from a pacemaker to a position inside a heart for stimulating the heart activity, comprising:
a helix (2) for attaching the cardiac stimulator lead (7) to a heart wall by screw rotating the helix (2) into the heart wall;
a shaft (4) on which the helix (2) is mounted; and
a tip tube (1) having on the inside a post (9) which protrudes between adjacent coils of the helix (2), whereby when rotating the shaft (4) by means of a rotary member, the helix (2) as well as the shaft (4) will be screw rotated outward or inward in the tip tube (1) by engagement between the helix (2) and the post (9),
wherein the shaft (4) is formed with a stop surface (12), such that when the stop surface (12) reaches the post (9), the screw rotating motion is interrupted by abutment of the stop surface (12) against the post (9), **characterized in that**
the stop surface (12) is oriented in a plane which is parallel to a radial direction of the shaft (4) and parallel to the longitudinal direction of the shaft (4) or in a plane which is parallel to a radial direction of the shaft (4) and has angle that is equal to the pitch angle of the helix (2) in relation to the longitudinal direction of the shaft (4).

2. A cardiac stimulator lead according to claim 1, **characterized in that** the stop surface (12) is oriented in a plane which is parallel to a radial direction of the shaft (4) and parallel to the longitudinal direction of the shaft (4).

3. A cardiac stimulator lead according to claim 1, **characterized in that** the stop surface (12) is oriented in a plane parallel to a radial plane of the shaft (4).

4. A cardiac stimulator lead according to any of the claims 1 to 3, **characterized in that** the stop surface (12) is a plane surface.

5. A cardiac stimulator lead according to any of the claims 1 to 4, **characterized in that** the stop surface (12) is formed on the shaft in an area between the two most proximal coils of the helix (2).

## Patentansprüche

1. Kardiale Stimulatorleitung (7) der Art, die zum Übertragen elektrischer Signale von einem Herzschrittmacher zu einer Position im Inneren eines Herzens zum Stimulieren der Herzaktivität verwendet wird, umfassend:
eine Spirale (2) zum Befestigen der kardialen Stimulatorleitung (7) an einer Herzwand durch Eindrehen der Spirale (2) in die Herzwand;
einen Schaft (4), auf dem die Spirale (2) montiert ist; und
eine Endröhre (1), die an der Innenseite eine Säule (9) aufweist, die zwischen benachbarten Windungen der Spirale (2) hervorsteht, wobei beim Drehen des Schafts (4) mittels eines Drehelements die Spirale (2) sowie der Schaft (4) in die Endröhre (1) durch Eingreifen zwischen der Spirale (2) und der Säule (9) nach außen oder innen geschraubt werden,
wobei der Schaft (4) mit einer Stoppfläche (12) gebildet ist, so dass, wenn die Stoppfläche (12) die Säule (9) erreicht, die Schraubdrehbewegung durch Anlage der Stoppfläche (12) an der Säule (9) unterbrochen wird, **dadurch gekennzeichnet, dass**
die Stoppfläche (12) in einer Ebene ausgerichtet ist, die parallel zu einer radialen Richtung des Schafts (4) und parallel zur Längsrichtung des Schafts (4) ist oder in einer Ebene, die parallel zu einer radialen Richtung des Schafts (4) ist und einen Winkel aufweist, der gleich dem Steigerungswinkel der Spirale (2) in Bezug auf die Längsrichtung des Schafts (4) ist.

2. Kardiale Stimulatorleitung nach Anspruch 1, **dadurch gekennzeichnet, dass** die Stoppfläche (12) in einer Ebene ausgerichtet ist, die parallel zu einer radialen Richtung des Schafts (4) und parallel zur Längsrichtung des Schafts (4) ist.

3. Kardiale Stimulatorleitung nach Anspruch 1, **dadurch gekennzeichnet, dass** die Stoppfläche (12) in einer Ebene parallel zur radialen Ebene des Schafts (4) ist.

4. Kardiale Stimulatorleitung nach einem der Ansprüche 1 bis 3, **dadurch gekennzeichnet, dass** die Stoppfläche (12) eine ebene Fläche ist.

5. Kardiale Stimulatorleitung nach einem der Ansprüche 1 bis 4, **dadurch gekennzeichnet, dass** die Stoppfläche (12) auf dem Schaft in einem Bereich zwischen den zwei proximalsten Windungen der Spirale (2) gebildet ist.

## Revendications

1. Dérivation (7) de stimulateur cardiaque, du type utilisé pour transmettre des signaux électriques d'un pacemaker à une position à l'intérieur d'un coeur pour stimuler l'activité du coeur, comprenant :
une hélice (2) pour attacher la dérivation (7) de stimulateur cardiaque à une paroi de coeur en vissant l'hélice (2) dans la paroi du coeur ;
un axe (4) sur lequel l'hélice (2) est montée ; et
un tube d'embout (1) présentant sur l'intérieur un montant (9) qui dépasse entre des spires adjacentes de l'hélice (2), ce par quoi, quand l'axe (4) est tourné au moyen d'un élément rotatif, l'hélice (2) ainsi que l'axe (4) seront vissés vers l'extérieur ou vers l'intérieur dans le tube d'embout (1) par engagement entre l'hélice (2) et le montant (9),
l'axe (4) étant formé avec une surface d'arrêt (12), de sorte que quand la surface d'arrêt (12) atteint le montant (9), le mouvement de vissage est interrompu par la butée de la surface d'arrêt (12) contre le montant (9), **caractérisée en ce que**
la surface d'arrêt (12) est orientée dans un plan qui est parallèle à une direction radiale de l'axe (4) et parallèle à la direction longitudinale de l'axe (4) ou dans un plan qui est parallèle à une direction radiale de l'axe (4) et présente un angle qui est égal à l'angle de pas de l'hélice (2) par rapport à la direction longitudinale de l'axe (4).

2. Dérivation de stimulateur cardiaque selon la revendication 1, **caractérisée en ce que** la surface d'arrêt (12) est orientée dans un plan qui est parallèle à une direction radiale de l'axe (4) et parallèle à la direction longitudinale de l'axe (4).

3. Dérivation de stimulateur cardiaque selon la revendication 1, **caractérisée en ce que** la surface d'arrêt (12) est orientée dans un plan parallèle à un plan radial de l'axe (4).

4. Dérivation de stimulateur cardiaque selon l'une quelconque des revendications 1 à 3, **caractérisée en ce que** la surface d'arrêt (12) est une surface plane.

5. Dérivation de stimulateur cardiaque selon l'une quelconque des revendications 1 à 4, **caractérisée en ce que** la surface d'arrêt (12) est formée sur l'axe dans une zone entre les deux spires les plus proximales de l'hélice (2).
